(19) Europäisches Patentamt
European Patent Office
Office européen des brevets

(11) **EP 1 568 377 A1**

(12) **EUROPEAN PATENT APPLICATION**
published in accordance with Art. 158(3) EPC

(43) Date of publication:
**31.08.2005 Bulletin 2005/35**

(51) Int Cl.7: **A61K 38/57**, A61P 1/02,
A61P 3/14, A61P 19/10,
A61P 31/04, A61P 31/12,
A61P 35/00, A61P 43/00

(21) Application number: **03812286.7**

(22) Date of filing: **10.11.2003**

(86) International application number:
**PCT/JP2003/014263**

(87) International publication number:
**WO 2004/050118 (17.06.2004 Gazette 2004/25)**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **29.11.2002 JP 2002347801
23.05.2003 JP 2003147035**

(71) Applicant: **MORINAGA MILK INDUSTRY CO., LTD.
Minato-ku, Tokyo 108-8384 (JP)**

(72) Inventors:
• **KATUNUMA, N.
TOKUSHIMA BUNRI UNI.,Inst.Health Sci.
Tokushima-shi, Tokushima 770-8514 (JP)**

• **YAMADA, Akio
MORINAGA MILK INDUSTRY CO., LTD.
Zama-shi, Kanagawa 228-8583 (JP)**
• **KAWAGUCHI, Y.
MORINAGA MILK INDUSTRY CO.,LTD
Zama-shi, Kanagawa 228-8583 (JP)**
• **TAKAKURA, Natsuko
MORINAGA MILK INDUSTRY CO., LTD.
Zama-shi, Kanagawa 228-8583 (JP)**

(74) Representative: **Bannerman, David Gardner et al
Withers & Rogers LLP
Goldings House,
2 Hays Lane
London SE1 2HW (GB)**

(54) **CYSTEINE PROTEASE INHIBITOR**

(57) This invention relates to a cysteine protease inhibitor comprising casein which is a protein derived from milk, a partial peptide of casein, and/or hydrolysate of casein as an active ingredient. The cysteine protease inhibitor of the present invention can be used as preventive or therapeutic drug for osteoporosis, malignant hypercalcemia, breast cancer, prostate cancer, periodontitis or bacterial and viral infectious diseases, and food, drink, feed and the like.

Fig. 3

EP 1 568 377 A1

**Description**

Technical Field

The present invention relates to a cysteine protease inhibitor comprising casein, a partial peptide of casein or a casein hydrolysate as an active ingredient, which can be used for preventive or therapeutic agents for osteoporosis, malignant hypercalcemia, breast cancer, prostate cancer, periodontitis, bacterial or viral infectious disease or the like, and used for food, drink, feed and the like.

Background Art

A proteolytic enzyme having a thiol group at its active center is generally named as cysteine protease (thiolprotease). Cathepsin L, cathepsin B or cathepsin K is one of typical cysteine proteases along with calcium-dependent neutral protease (CAMP), papain, ficin, promelain and the like. Substances having inhibitory action against those cysteine proteases are expected to be used as therapeutic agents for diseases associated with cysteine proteases including muscular dystrophy, dystrophia, myocardial infarct, apoplexy, Alzheimer's disease, disturbance of consciousness or motility caused by head trauma, multiple sclerosis, peripheral neuropathy, cataract, inflammation, allergy, fulminant hepatitis, osteoporosis, hypercalcemia, breast cancer, prostate cancer or enlarged prostate, or as a growth inhibitor or metastasis preventive agent for cancer, an antithrombotic drug and the like. Moreover, in recent years, the relationship between cathepsin L, cathepsin B, and osteoporosis or malignant hypercalcemia has been elucidated based on the studies made by Katsunuma et al. Accordingly, in particular, application of a cathepsin L inhibitor as a therapeutic agent for osteoporosis or malignant hypercalcemia is attracting attention (Nobuhiko Katsunuma, "BIO media" Vol. 7, No. 6, pp. 73 to 77, 1992). In bone tissue, osteogenesis by osteoblast and bone resorption by osteoclast are occurring throughout life. In growing ages, bone mass is increased due to excessive osteogenesis over bone resorption, whereas in older ages, bone mass is decreased due to excessive bone resorption over osteogenesis, which leads to development of osteoporosis. There are various causes of osteoporosis, and especially, one of main causes thereof is bone collapse (bone resorption), which is further divided into the following two causes. That is, one is attributed to failure of absorption and deposition of calcium, more specifically related to supply, transfer, absorption and deposition of calcium, in which vitamin D derivatives, female hormone (estrogen) and the like are considered to be involved. The other one is associated with promoted degradation of collagen, a bone-supporting tissue, and a principal cause thereof is degradation of bone collagen by a cysteine protease which is secreted from the lysosome located in the osteoclast, in particular, cathepsin L, cathepsin B, and cathepsin K. The cathepsin L and capthepsin B secreted from the lysosome in the osteoclast promote degradation of collagen in bone tissue, whereby old bones are caused to lyse, and calcium is freed and released into the blood together with hydroxyproline. Therefore, inhibition of collagen degradation by cathepsin L, cathepsin B and cathepsin K enables prevention of excessive bone collapse, thus making it possible to treat osteoporosis. Estrogen, anabolic hormone, calcium preparation, vitamin D, calcitonin, bisphosphonate or the like is known as a therapeutic agent for such osteoporosis. In addition, development of a therapeutic agent for osteoporosis using some cysteine protease inhibitors is under progress as regards a therapeutic agent for osteoporosis having a mechanism of action of so-called cysteine protease inhibition including cathepsin L inhibition, cathepsin B inhibition and cathepsin K inhibition (JP 07-179496 A, JP 2002-501502A). However, further development of therapeutic agents for osteoporosis has been desired.

Meanwhile, hypercalcemia is a metabolic disorder where calcium concentration in the serum is elevated beyond the normal value, and is often seen in patients with tumor. It is said that, if hypercalcemia is neglected, the life of the patients would be 10 days long at most. In many cases, it is caused due to bone metastasis of tumor. When tumor transfers to the bone, bone collapse occurs, and calcium is released into the blood. The released calcium is disposed in the kidney, and hypercalcemia develops when a speed of bone collapse exceeds the disposing capacity of the kidney. As a method of treatment, a method of promoting calcium excretion from the kidney by use of infusion of physiological saline with furosemide and a method of using calcitonin as a therapeutic agent for osteoporosis are known. Namely, it is said that a therapeutic agent for osteoporosis which suppresses bone resorption can be also effective as a therapeutic agent for malignant hypercalcemia.

The followings have already been disclosed as cysteine protease inhibitors which may be used for such purposes, by the inventors of the present invention.

    (1) Cathepsin L-specific inhibitory polypeptide (JP 07-179496 A)
    (2) Thiol protease inhibitor (JP 09-221425 A)
    (3) Valine derivative and its use (JP 2001-139534 A)
    (4) Thiol protease inhibitor (JP 07-242600 A)
    (5) FA-70C1 substance (JP 2000-72797 A)

(6) FA-70D substance, its production method and its use (WO 97/31122)

However, development of a more versatile cysteine protease inhibitor has been desired from the viewpoint of usage as food material.

On the other hand, it has been known so far that protease inhibitory substances are present in breast milk. The known protease inhibitory substances contained in breast milk include α1-antichymotrypsin and α1-antitrypsin, and inhibitors including inter α2-trypsin inhibitory substance, α2-antiplasmin, α2-macroglobulin, antithrombin III, antileukoprotease are contained in a minute amount in breast milk (Isao Kiyosawa, "Human Milk in Infant Nutrition," Kanehara & Co., Ltd., pp. 80 to 81).

The following proteins having cysteine protease inhibitory activity in milk have already been disclosed.

(1) Novel cysteine protease inhibitor with a molecular weight of approximately 57 kDa which has a sugar chain and is derived from bovine colostrum (JP 07-2896 A).
(2) Novel cysteine protease inhibitor with a molecular weight of 16 ± 2 kDa or 13 ± 2 kDa which is derived from colostrum (JP 07-126294 A).
(3) Novel protein with a molecular weight of 16 ± 2 kDa or 13 ± 2 kDa which is derived from human colostrums, and a production method for the same (JP 10-80281 A).
(4) Bone resorption inhibitor which includes basic cystatin of milk origin which is prepared from milk and/or degradation product of basic cystatin of milk origin, as an active ingredient (JP 2000-281587 A).
(5) Cystatin C contained in milk basic protein (MBP), and a bone resorption inhibitory activity by the cystatin C *in vitro* ("Bioscience, Biotechnology, and Biochemistry, Japan" Vol. 66, No. 12, 2002, pp. 2531 to 2536).

Proteins contained in mammalian milk in a large amount include lactoferrin and β-casein. Caseins are classified into αs-casein, β-casein and κ-casein. Casein in human milk is almost β-casein, αs-casein not being present or being present only in trace amount, whereas casein in bovine milk is composed of almost equal amounts of αs-casein and β-casein. In addition to a function as nutritional component, casein attracts attentions in recent years because a bioactive peptide having a calcium absorption-stimulatory effect or macrophage phagocytosis-activating effect which is inherently included in the primary structure of the protein has been found. In addition to its high nutritional value, casein contributes to our dietary life as raw material of milk products, or as component of a variety of foods such as cheese, yogurt and skim milk.

As an invention that utilizes casein, the present applicant discloses a preventive agent for arteriosclerosis which comprises κ-casein or a hydrolysate of κ-casein as an active ingredient (JP 08-81388 A).

In addition, it has been disclosed that β-casein isolated from human milk or its recombinant form, or a hydrolysate of either of them has an inhibitory activity on attachment of Haemophilus influenzae to human cells (JP10-500101 A), and an inhibitory effect on infection of RS virus (Respiratory Syncytial Virus) to mammalian cells (JP10-500100 A).

Moreover, angiotensin converting enzyme inhibitory activity of a β-casein hydrolysate has been disclosed (JP 06-128287 A and JP 06-277090 A).

However, it is not known that casein and a partial peptide thereof have cysteine protease inhibitory action.

Disclosure of the Invention

An object of the present invention is to provide a versatile cysteine protease inhibitor which can be widely used as a foodmaterial, and which can be used for preventive or therapeutic agents for osteoporosis, malignant hypercalcemia, breast cancer, prostate cancer, periodontitis, or bacterial and viral infectious diseases and the like, and for various types of food, drink and feed.

As a result of extensive studies for searching a cysteine protease inhibitor which may be used as antigenic-free and safe material, the inventors of the present invention found that casein which is a protein of milk origin, a partial peptide of casein and a casein hydrolysate have cysteine protease inhibitory activity, and thereby completed the present invention.

The gist of the present invention is as in the following (1) to (24).

(1) A cysteine protease inhibitor comprising casein or a partial peptide thereof as an active ingredient.
(2) The cysteine protease inhibitor according to (1), wherein the casein or the partial peptide thereof is derived from human or bovine.
(3) A cysteine protease inhibitor comprising casein as shown in the following (A) or (B) or a partial peptide thereof as an active ingredient;

(A) a peptide having amino acid sequence of at least amino acid numbers 133 to 151 of the amino acid sequence shown in SEQ ID No. 1 of the Sequence Listing, or

(B) a peptide having amino acid sequence of at least amino acid numbers 133 to 151 of the amino acid sequence shown in the SEQ ID No. 1 of the Sequence Listing, including substitution, deletion, insertion, addition or inversion of one or plural amino acids, and having cysteine protease inhibitory activity.

(4) A cysteine protease inhibitor comprising casein as shown in the following (C) or (D) or a partial peptide thereof as an active ingredient;

(C) a peptide having amino acid sequence of at least amino acid numbers 142 to 160 of the amino acid sequence shown in SEQ ID No. 2 of the Sequence Listing, or

(D) a peptide having amino acid sequence of at least amino acid numbers 142 to 160 of the amino acid sequence shown in the SEQ ID No. 2 of the Sequence Listing, including substitution, deletion, insertion, addition or inversion of one or plural amino acids, and having cysteine protease inhibitory activity.

(5) A cysteine protease inhibitor comprising casein hydrolysate, which is obtainable by hydrolyzing casein with protease and has cysteine protease inhibitory function, as an active ingredient.

(6) The cysteine protease inhibitor according to (5), wherein the protease is one or a plurality of proteases selected from the group consisting of proteases derived from animals and proteases derived from microorganisms.

(7) The cysteine protease inhibitor according to (5) or (6), wherein the degree of hydrolysis of the casein hydrolysate is 6 to 45%.

(8) The cysteine protease inhibitor according to any one of (5) to (7), wherein number-average molecular weight of the casein hydrolysate is 200 to 5,000 dalton.

(9) The cysteine protease inhibitor according to any one of (5) to (8), which comprises the casein hydrolysate not less than 0.005% by mass with respect to the total amount.

(10) The cysteine protease inhibitor according to any one of (1) to (9), wherein the cysteine protease inhibitor is a preventive or therapeutic agent for a disease associated with cysteine protease.

(11) The cysteine protease inhibitor according to (10), wherein the disease associated with the cysteine protease is osteoporosis, malignant hypercalcemia, breast cancer, prostate cancer, periodontitis, bacterial and viral infectious diseases or the like.

(12) A food and drink composition or feed composition, which is produced by adding the cysteine protease inhibitor according to any one of (1) to (11).

(13) A method for treating a disease associated with cysteine protease, wherein the cysteine protease inhibitor according to any one of (1) to (11) is administered to a subject.

(14) A use of casein or a partial peptide thereof in manufacture of a cysteine protease inhibitor.

(15) The use according to (14), wherein the casein or the partial peptide thereof is derived from human or bovine.

(16) A use of casein as shown in the following (A) or (B) or a partial peptide thereof in manufacture of cysteine protease inhibitor;

(A) a peptide having amino acid sequence of at least amino acid numbers 133 to 151 of the amino acid sequence shown in SEQ ID No. 1 of the Sequence Listing, or

(B) a peptide having amino acid sequence of at least amino acid numbers 133 to 151 of the amino acid sequence shown in SEQ ID No. 1 of the Sequence Listing, including substitution, deletion, insertion, addition or inversion of one or plural amino acids, and having cysteine protease inhibitory activity.

(17) A use of casein as shown in the following (C) or (D) or a partial peptide thereof in manufacture of cysteine protease inhibitor;

(C) a peptide having amino acid sequence of at least amino acid numbers 142 to 160 of the amino acid sequences shown in SEQ ID No. 2 of the Sequence Listing, or

(D) a peptide having amino acid sequence of at least amino acid numbers 142 to 160 of the amino acid sequences shown in SEQ ID No. 2 of the Sequence Listing, including substitution, deletion, insertion, addition or inversion of one or plural amino acids, and having cysteine protease inhibitory activity.

(18) A use of a casein hydrolysate, which is obtainable by hydrolyzing casein with a protease and has cysteine protease inhibitory function, in manufacture of a cysteine protease inhibitor.

(19) The use according to (18), wherein the protease is one or a plurality of proteases selected from the group consisting of proteases derived from animals and proteases derived from microorganisms.

(20) The use according to (18) or (19), wherein the degree of hydrolysis of the casein hydrolysate is 6 to 45%.

(21) The use according to any one of (18) to (20), wherein number-average molecular weight of the casein hydrolysate is 200 to 5,000 dalton.

(22) Theuseaccordingtoanyoneof (18) to (21), which comprises the casein hydrolysate not less than 0.005% by mass with respect to the total amount.

(23) The use according to any one of (14) to (22), wherein the cysteine protease inhibitor is a preventive or therapeutic agent for a disease associated with cysteine protease.

(24) The use according to (23), wherein the disease associated with cysteine protease is osteoporosis, malignant hypercalcemia, breast cancer, prostate cancer, periodontitis, bacterial and viral infectious diseases, or the like.

Brief Description of the Drawings

Fig. 1 is a diagram (photograph) which shows detection of bovine milk protein by reverse zymography.
Fig. 2 is a diagram which shows amino acid sequences of bovine β-casein and bovine β-casein peptide.
Fig. 3 is a diagram which shows cysteine protease inhibitory activities of caseins to papain.
Fig. 4 is a diagram which shows spectrum of cysteine protease inhibitory activity of β-casein.
Fig. 5 is a diagram which shows amino acid sequences of human β-casein and human β-casein peptide.

Best Mode for carrying out the Invention

Preferred embodiments of the present invention will be described in detail below. However, the present invention is not limited to the following preferred embodiments, and any changes may be made within the scope of the present invention. All percentages used in the present specification are represented by weight unless otherwise noted.

The present invention relates to a cysteine protease inhibitor comprising casein, partial peptide of casein or casein hydrolysate as an active ingredient. Casein used in the present invention may be various kinds of commercially available casein, casein isolated from milk of human, bovine, horse or goat by a conventional procedure (for example, isoelectric precipitation procedure), or casein produced by use of gene recombination techniques and the like. Casein is classified into α-casein, β-casein, and κ-casein and any type of casein may be used for the present invention, and β-casein or κ-casein is preferably used. Above all, β-casein derived from human (for example, β-casein derived from human having the amino acid sequence described in Swiss-Prot Accession No. : P05814) and β-casein derived from bovine (for example, bovine β-casein having the amino acid sequence described in Swiss-Prot Accession No: P02666) are preferable. Specifically, the amino acid sequence of the human β-casein is shown in SEQ ID No. 1, and the amino acid sequence of the bovine β-casein is shown in SEQ ID No. 2.

The partial peptide of casein used in the present invention may be obtained by hydrolyzing the casein with an acid or protease according to a known method, and purifying the resultant partial peptide. For instance, the partial peptide may be produced by hydrolysing casein with lysyl endopeptidase in a 100 mM Tris-HCl buffer solution (pH 8.5) at 35°C for three or longer hours, and purifying the resultant partial peptide by a high performance liquid chromatography (HPLC) method etc.

Preferred embodiments of casein or a partial peptide of casein which can be used in the present invention include human β-casein having the amino acid sequence shown in SEQ ID No. 1, or a peptide having amino acid sequence of at least amino acid numbers 133 to 151 of the amino acid sequence shown in SEQ ID No. 1. In addition, bovine β-casein having the amino acid sequence shown in SEQ ID No. 2 or a peptide having amino acid sequence of at least amino acid numbers 142 to 160 of the amino acid sequence shown in SEQ ID No. 2 can be exemplified. Note that sequences of amino acid numbers 1 to 15 of the amino acid sequence shown in SEQ ID No. 1 of the Sequence Listing and the amino acid sequence shown in SEQ ID No. 2 of the Sequence Listing are signal sequences.

The casein and partial peptide of casein have cysteine protease inhibitory activity, and therefore they can be used for the cysteine protease inhibitor of the present invention. Moreover, it is considered that peptides having amino acid sequence which includes amino acid numbers 133 to 151 of SEQ ID No. 1 in the Sequence Listing and further extends to one or both of the N-termius side and the C-termius side, and peptides having amino acid sequences which includes amino acid numbers 142 to 160 of SEQ ID No. 2 in the Sequence Listing and further extends to one or both of the N termius side and the C termius side, have cysteine protease inhibitory activity, because full-length casein has cysteine protease inhibitory activity.

Since the present invention have revealed a domain showing cysteine protease inhibitory activity, the above peptides may be obtained by chemical synthesis based on amino acid sequence including the domain, as well as by gene recombination techniques. For example, appropriate primers are prepared on the basis of the nucleotide sequence coding for amino acid sequence including the domain. The nucleotide sequence is then amplified by PCR and the like using the primers and cDNA including the target nucleotide sequence as a template. The obtained nucleotide sequence is expressed using an appropriate expression system, thereby the peptides described above can be obtained.

In general, there exist mutations including substitution, deletion, insertion, addition or inversion of one or more nucleotides at one or more positions in a gene, due to difference in species, genus, individual and the like, and mutation arises in an amino acid of a protein encoded by a gene including the above mutation. Casein and a partial peptide of casein which can be used for the present invention may thus include such mutation within a range that the cysteine protease inhibitory activity is not impaired. Casein or a partial peptide of casein which can be used for the present invention includes a peptide which has amino acid sequence of at least amino acid numbers 133 to 151 of the amino acid sequence shown in SEQ IDNo. 1 of the Sequence Listing and includes substitution, deletion, insertion, addition or inversion of one or plural amino acids and has cysteine protease inhibitory activity, and a peptide which has amino acid sequence of at least amino acid numbers 142 to 160 of the amino acid sequence shown in SEQ ID No. 2 of the Sequence Listing and includes substitution, deletion, insertion, addition, or inversion of one or plural amino acids and has cysteine protease inhibitory activity. The term plural, as used herein, varies with a position or a kind of an amino acid residue in the protein conformation of amino acid numbers 133 to 151 of the amino acid sequence shown in SEQ ID No. 1 of the Sequence Listing, or amino acid numbers 142 to 160 of the amino acid sequence shown in SEQ ID No. 2 of the Sequence Listing, and for example, it refers to 2 to 5 amino acids, preferably 2 or 3 amino acids.

Further, substitution, deletion and the like of one or plural amino acids may be included in amino acids except the amino acids of amino acid numbers 133 to 151 of the amino acid sequence shown in SEQ ID No. 1 of the Sequence Listing, or amino acids except the amino acids of amino acid numbers 142 to 160 of the amino acid sequence shown in SEQ ID No. 2 of the Sequence Listing. In this case, for example, the term plural refers to 2 to 10 amino acids, preferably 2 to 5 amino acids, though varying with a position or a kind of an amino acid residue in protein conformation.

Moreover, examples of casein or a partial peptide of casein which can be used for the present invention include a protein or a peptide which has homology not less than 80%, preferably not less than 90%, more preferably not less than 95% in an amino acid sequence with a protein or a peptide having amino acid sequence of at least amino acid numbers 133 to 151 of the amino acid sequence shown in SEQ ID No. 1 of the Sequence Listing, or protein or a peptide having amino acid sequence of at least amino acid numbers 142 to 160 of the amino acid sequence shown in SEQ ID No. 2 of the Sequence Listing, and which has cysteine protease inhibitory activity.

Nucleotide sequence which codes for a protein or a peptide substantially identical with the casein protein or peptide as described above can be obtained, for example, by modifying the nucleotide sequence with site-directed mutagenesis in such a manner that an amino acid residue located at a specific site includes substitution, deletion, addition or inversion. The modified nucleotide sequence may be obtained by means of conventional mutagenesis treatment. Nucleotide sequence which codes for protein or a peptide substantially identical with casein or a casein peptide may be obtained by expressing a nucleotide sequence including mutation in appropriate cells and examining cysteine protease inhibitory activity by a method of determining the cysteine protease inhibitory activity described in Examples of the present invention.

Hydrolysate of casein may also be used in the present invention. Caseins which can be hydrolyzed include those described above. Casein hydrolysate can be obtained by hydrolyzing those caseins with protease as described below.

That is, the material casein as described above is dispersed and dissolved in water or hot water. Although concentration of the solution is not particularly limited, it is usually desirable to keep a concentration range of around 5 to 15% of protein from the viewpoint of efficiency and operability of hydrolysis. From the viewpoint of preventing rancid due to contamination, it is desirable to sterilize the obtained solution containing the casein by heating at 70 to 90°C for 15 seconds to 10 minutes. Next, it is preferable to add alkaline reagent or acid reagent into the solution containing the casein and to adjust pH to optimal pH of protease or its vicinity. Alkaline reagent and acid reagent used for the method of the present invention may be any alkaline reagent or acid reagent as far as they are acceptable for foods or pharmaceutical products. Specific examples of alkaline reagents include sodium hydroxide, potassium hydroxide and potassium carbonate, whereas examples of acid reagents include hydrochloric acid, citric acid, phosphoric acid and acetic acid.

After that, a protease is added to the casein solution. The protease is not particularly limited as far as it is an enzyme which can hydrolyze protein, and preferably is an enzyme derived from animals or microorganisms. In addition, an enzyme is preferably endopeptidase. As an endopeptidase, various enzymes such as pancreatin, pepsin, trypsin, elastase and the like can be used. The term "derived from" means that the above-mentioned organisms possess it by nature, but does not mean a source from which it is collected. For example, the protease obtained by introducing a gene coding for a protease produced by Bacillus subtilis into Escherichia coli and expressing the gene, is "derived from" Bacillus subtilis.

It is preferable that protease is added at a rate of 20 to 200 units of activity/g of casein (this unit will be described later). Here, the unit of activity can be determined, for example, by the following method: powder containing protease is dispersed or dissolved in 0.1 mol phosphate buffer solution (pH 7.0) at a rate of 0.2g/100 ml to prepare an enzyme solution. Meanwhile, leucylparanitroanilide (manufactured by Kokusan Chemical Co. , Ltd., hereinafter, referred to as Leu-pNA) is dissolved in 0.1mol phosphate buffer solution (pH 7.0) to prepare 2 mM of substrate solution. 1 ml of substrate solution is added to 1 ml of an enzyme solution to allow reaction at 37°C for 5 minutes, followed by addition of 2 ml of 30% acetic acid solution to terminate the reaction. The reaction solution is then filtered with a membrane-filter and absorbance of the filtered solution is measured at a wavelength of 410 nm. The activity unit of protease, while the amount of an enzyme required to degrade 1 μmol of Leu-pNA for 1 minute is defined as 1 activity unit, can be calculated according to the following formula.

$$\text{Activity unit (per 1 g of powder)} = 20 \times (A/B)$$

In the above formula, A and B represent the absorbance of a sample or 0. 25 mM paranitro aniline at a wavelength of 410 nm, respectively.

The protease used in the present invention may be of one kind or of two or more kinds. When two or more kinds of enzymes are used, enzyme reactions may be carried out either simultaneously or separately.

A solution to which an enzyme is added is maintained at suitable temperature, for example, at 30 to 60°C, preferably at 45 to 55°C in accordance with the type of the enzyme, and then casein hydrolysis is initiated. The hydrolysis reaction is allowed to be continued until a preferable degree of hydrolysis is achieved, while monitoring the degree of hydrolysis of the enzyme reaction. In the present invention, the degree of hydrolysis of a casein hydrolyate is particularly preferably 6 to 45%. Here, the degree of hydrolysis of the casein hydrolysate not less than 6% suggests that the degradation further proceeds. That is, the degree of hydrolysis is preferably not less than 6%, since the degree of hydrolysis below 6% suggests a possibility that undegraded casein which is not subjected to the enzyme reaction still remains.

The method for calculating a degree of hydrolysis of protein is: for example, total nitrogen in a sample is measured with the Kj eldahl method (The Japanese Society for Food Science and Technology edition, "Shokuhin bunsekiho," p102, Korin Publishing Co., Ltd., 1984), and formol nitrogen in a sample is measured with a formol titration method (Ikeda et al. edited, "Food Engineering Experiments," Vol. 1, p547, Yokendo Co. Ltd., 1970) and, based on those measurement values, the degree of hydrolysis is calculated according to the following formula.

$$\text{Degree of hydrolysis (\%)} = (\text{formol nitrogen}/\text{total nitrogen}) \times 100$$

Termination of hydrolysis reaction is performed by inactivating enzymes in a hydrolysis solution, and may be carried out by inactivation with conventional heat treatment. Heating temperature and retention time of the inactivation with heat treatment can be set a condition under which enzyme is sufficiently inactivated in consideration of the thermal stability of the enzyme used. For example, heat treatment can be performed at temperature ranging from 80 to 130°C for retention time of 2 seconds to 30 minutes. The obtained reaction solution may be adjusted within a range of pH 5.5 to 7 by use of acids such as citric acid when needed.

In this specification, the term "number-average molecular weight" refers to an average value of the molecular weight of a macromolecule compound based on different indexes, as described in a literature concerning the number-average molecular weight (edition by The Society of Polymer Science, Japan "The Foundation of Polymer Science" pp. 116 to 119, Tokyo Kagaku Dozin Co., Ltd., 1978). That is, macromolecule compounds such as protein hydrolysate are heterogeneous substances, and have distribution in molecular weight, and therefore the molecular weight of the protein hydrolysate needs to be represented by the average molecular weight to treat physicochemically. The number-average molecular weight (hereinafter, sometimes abbreviated to Mn) is an average with regard to number of molecules. Provided that a molecular weight of a peptide chain i is represented by Mi and the number of molecules thereof is represented by Ni, the number-average molecular weight is defined by the following general formula I.

[General Formula I]

$$Mn = \sum_{i=1}^{\infty} MiNi \bigg/ \sum_{i=1}^{\infty} Ni$$

When the number-average molecular weight is determined in the present invention, it can be calculated by determining molecular weight distribution with the high performance liquid chromatography, and analyzing the data from analytical curves with GPC analysis system. A specific condition for the high performance liquid chromatography can be mentioned as a condition where Poly Hydroxyethyl Aspartamide Column (manufactured by Poly LC, Inc., 4.6 mm x 400 mm) is used as a column and elution is performed with 20 mM sodium chloride and 50 mM formic acid at an elution rate of 0.5 ml/min. Molecular weight is determined with a UV detector (manufactured by Shimadzu Corporation, wavelength of 215 nm) and an analytical curve is created from a sample with known molecular weight, and data is analyzed with the GPC analysis system (manufactured by Shimadzu Corporation, wavelength of 215 nm), thereby the number-average molecular weight can be calculated.

In the present invention, the number-average molecular weight of a casein hydrolysate is particularly preferably 200 to 5,000 dalton. Here, the number-average molecular weight is preferably not more than 5,000 dalton, because undegraded casein which is not subjected to hydrolysis reaction is not included and thus a casein hydrolysate, an active ingredient in the present invention, is considered to be more certainly obtained when the number-average molecular weight of the casein hydrolysate is not more than 5,000 dalton.

The obtained solution containing casein hydrolysate may be directly used, and may also be used as concentrated solution which is concentrated by conventional methods, or as powder obtained by drying the concentrated solution by conventional method.

The casein hydrolysate as obtained above has a cysteine protease inhibitory activity. Accordingly, a condition for producing a casein hydrolysate can be appropriately set by using a cysteine protease inhibitory activity as an index.

In the protease inhibitor of the present invention, casein, a partial peptide of casein, or a casein hydrolysate may be used alone, or two or more thereof may be used together. Moreover, one type of a partial peptide or a hydrolysate of casein may be used alone, or plural types thereof may be used in combination.

Casein, a partial peptide of casein, or hydrolysate of casein which can be used for the present invention has inhibitory activity against cysteine proteases such as cathepsin B, L and papain. The cysteine protease inhibitory activity can be determined in accordance with the methods of Barrett et al (Methods in Enzymology, Vol. 80, pp. 535-561, 1981). For example, Z-Phe-Arg-MCA (Benzyloxycarbonyl-L-Phenylalanyl-L-Arginine4-Methyl-Coumaryl-7-Amide: final concentration of 20 mM: manufactured by Peptide Institute, Inc.) is added as a substrate into a solution in which casein, a partial peptide and/or a hydrolysate of casein is dissolved in 0.1 M acetic acid buffer (pH 5.5), and then a cysteine protease (papain in the present Examples: manufactured by Sigma Co., Ltd.) solution (final concentration: 15 units/ml) is added and the whole solution is mixed to allow reaction at 37°C for 10 minutes. After that, fluorescence intensity (excitation wavelength: 370 nm, emission wavelength: 460 nm) of AMC (7-Amino-4-Methyl-Coumarin) released from the digested substrate is measured with a fluorescence spectrometer (manufactured by Hitachi, Ltd.).

The cysteine protease inhibitor of the present invention can be produced by using casein, a partial peptide of casein and/or a casein hydrolysate, and combining them with a pharmaceutical acceptable carrier. A form of administration unit of pharmaceutical of the preparation in the present invention is not particularly limited, and may be appropriately selected in accordance with therapeutic purposes. Specifically, examples of the form of administration unit include a tablet, a pill, powder, liquid, a suspension, an emulsion, granules, capsule, syrup, a suppository, an ointment, and a patch. Additives such as a vehicle, a bonding agent, a disintegrator, a lubricant, a stabilizer, a flavoring agent, diluent, surfactant and a solution for injection, which are commonly used for normal pharmaceuticals as a pharmaceutical carrier, may be used upon preparation.

The amount of casein, a partial peptide of casein and/or a casein hydrolysate contained in the pharmaceutical of the present invention is not particularly limited and may be appropriately selected. For example, it may be usually set to 0.005 to 80% by mass, preferably 0.05 to 60% by mass in the preparation.

Diseases associated with cysteine protease can be treated by oral or parenteral administration of the cysteine protease inhibitor of the present invention to a subj ect. The term "subject" used herein may be either human beings or mammal other than human. A method of administering the pharmaceutical of the present invention is not particularly limited, and can be determined according to a form of the pharmaceutical, a subject's age or sex, and other conditions including a degree of the subject's symptom. A dosage of an active ingredient of the pharmaceutical of the present invention can be appropriately set based on a dose regimen, a subject's age or sex, a degree of a disease, and other conditions. Usually, the amount of casein, a partial peptide of casein and/or a casein hydrolysate as active ingredients may be set based on the amount ranging from 0.1 to 1,200 mg/kg/day, preferably 10 to 500 mg/kg/day, and the pharmaceutical may be administered once or plural times per day.

The cysteine protease inhibitor of the present invention is useful as preventive and therapeutic agents for diseases associated with cysteine protease, such as, allergy, muscular dystrophy, myocardial infarct, apoplexy, Alzheimer's disease, multiple sclerosis, cataract, osteoporosis, malignant hypercalcemia, enlarged prostate, breast cancer, prostate cancer, and periodontitis, or as an inhibitor of cancer cell growth or metastasis, or as a growth inhibitor of bacteria (Staphylococcus aureus V8, etc.) or viruses (poliovirus, herpesvirus, coronavirus, AIDS virus, etc.). The cysteine protease inhibitor of the present invention may be used alone, or used in combination with known preventive and therapeutic agents for the above-mentioned diseases or known growth inhibitor of the bacteria or viruses. Such combination can enhance preventive and therapeutic effects against the diseases, or a growth inhibitory effect against the bacteria or viruses. The known preventive and therapeutic agents for the above-mentioned diseases, or the growth inhibitor of the bacteria or viruses to be combined may be contained in the inhibitor of the present invention as an active ingredient, or may be commercialized as another agent and combined upon use without being contained in the inhibitor of the present invention.

The food and drink composition of the present invention may be produced by adding casein, a partial peptide of casein, and/or a casein hydrolysate to raw materials of food or drink, and it can be orally ingested. As the raw materials, those employed in drink or food may be generally used. The food and drink composition of the present invention may be prepared in a similar way as usual food and drink composition except that the cysteine protease inhibitor is added. Examples of the forms of the food and drink composition include drinks such as cold drink, carbonated drink, nutrition drink, fruit drink and lactobacillus beverage (including concentrated stock solution and adjustment powder of those drinks) ; ice sweets such as an ice cream, sherbet and shaved ice; confectionery such as candy, chewing gum, gum, chocolate, confectionery pill, snack food, biscuit, jelly, jam, cream and baked confectionery; milk products such as processed milk, milk beverage, fermented milk and butter; bread; enteral nutrition formula, liquid formula, infant formula and sports drink; and other functional food.

The amount of casein, a partial peptide of casein and/or a casein hydrolysate to be added in the food and drink composition of the present invention is properly set according to the form of the food and drink composition, and normally they may be added in an amount of 0.005 to 80% by mass, preferably 0.05 to 60% by mass in the food or drink.

The feed composition of the present invention can be produced by adding casein, a partial peptide of casein and/or a casein hydrolysate to feed, and orally administered to general mammals, livestock, pisciculture, farmed fish, and pet animals. Examples of the forms of feed composition include pet foods, livestock feed, and pisciculture feed, and the feed composition of the present invention can be produced by formulating casein with grains, lees, rice bran, fish flour, bone manure, oils and fats, skim milk, whey, mineral feed, yeast and the like.

The amount of casein, a partial peptide of casein, and/or a casein hydrolysate to be added in the feed composition of the present invention is properly set according to the form of the feed composition, and normally they may be added in an amount of 0. 005 to 80% by mass, preferably 0.05 to 60% by mass in the feed composition.

The food and drink composition or the feed composition of the present invention may be a food and drink composition or feed composition having indication of its efficacy as preventive or treatment of the diseases shown below. That is, it can be indicated as a preventive or treatment of diseases associated with cysteine protease, such as osteoporosis, malignant hypercalcemia, breast cancer, prostate cancer, periodontitis or bacterial and viral infectious diseases.

The term "indicated" used herein means informing the users of the above-mentioned efficacy, and includes, for example, indicating the above-mentioned efficacy on commercial products of the food and drink composition or the feed composition of the present invention or on packages or advertisement thereof, and transferring, turning over and displaying the substances having such indication. In particular, an embodiment in which it is indicated as a food for specified health uses [refer to Article 12(1), 5 of the regulation of Health Enforcement Law (April 30, 2003, Ordinance No. 86 of the Japanese Ministry of Health, Labor and Welfare)] is preferable.

Examples of producing a partial peptide of casein or a casein hydrolysate used as an active ingredient of the cysteine protease inhibitor of the present invention will be shown below.

[Production Example 1]

Peptide having amino acid sequence of amino acid numbers 133 to 151 in the amino acid sequence described in SEQ ID No. 1 was produced according to the following methods.

A peptide of the present invention was produced by synthesizing it with an automatic peptide synthesizer (manufactured by Applied Biosystems Co., Ltd., Model 433A).

Fmoc-group, an amino protective group of HMP resin (manufactured by Applied Biosystems Co. , Ltd.) which is a solidified resin for peptide synthesis, was cleaved with N-methylpyrrolidone (manufactured by Applied Biosystems Co., Ltd., hereinafter, abbreviated to NMP) containing 20% of piperidine, and the resin was washed with NMP. Then, Fmoc-threonine [specifically, Fmoc-amino acid (manufactured by Applied Biosystems Co., Ltd.) corresponding to the C-terminal amino acid of a peptide to be synthesized] was condensed to the resin using FastMoc (registered trademark) reagent kit (Applied Biosystems Co. , Ltd.), and the resin was washed with NMP. Next, the Fmoc group was cleaved again, and Fmoc-alanine corresponding to the second amino acid from the C-terminus was condensed, followed by washing the resin. Protective peptide resin was prepared by further repeating condensation of Fmoc-amino acid and washing, and crude peptide was recovered from the resin.

Peptides were purified from the crude peptides using high performance liquid chromatography (hereinafter, abbreviated to HPLC). An example of a column to be used includes a reverse phase C18-ODS (manufactured by Merck & Co, Inc., Lichrospher 100). The obtained purified peptides were analyzed by HPLC to further confirm that the purified product is a single peptide. The amino acid sequence of the purified peptide was determined by use of a gas-phase automatic amino acid sequencer (manufactured by Applied Biosystems Co., Ltd., Model 473A), and it was found to have amino acid sequence of amino acid numbers 133 to 151 in the SEQ ID No. 1.

According to the similar method, a peptide having amino acid sequence of amino acid numbers 142 to 160 in the amino acid sequence described in SEQ ID No. 2 was produced.

[Production Example 2]

100 g of commercial milk casein "Alacid" (protein content is 90%, manufactured by Newzealand Milk Products) was suspended at a concentration of 10% in purified water heated to 60°C, and dissolved completely by adding 2.5 g of sodium hydroxide. The solution was then sterilized at 85°C for 10 minutes, and the temperature of the solution was adjusted to 50°C. After that, 2,500U of pancreatin (manufactured by Amano Enzyme Co., Ltd., 112,000 U/g) were added as protease and hydrolysis was performed by keeping at 50°C for 4 hours. After denaturing the enzyme by heat-treatment at 90°C for 10 minutes, about 100 g of a casein hydrolysate was obtained by lyophilizing the solution. The obtained casein hydrolysate had a degree of hydrolysis of 9.5% and a number-average molecular weight of 910 dalton.

[Production Example 3]

100 g of commercially-available sodium casein "Alanate" (protein content is 90%, manufactured by Newzealand Milk Products) was dissolved at a concentration of 12% in purified water heated to 50°C. Subsequently, the solution was sterilized at 85°C for 10 minutes, and then adjusted to 40°C. Then, 250U of porcine trypsin (PTN6.0S; manufactured by Novozyme Inc., 1,250U/g) were added as protease and hydrosis was performed by keeping at 40°C for 6 hours. After denaturing the enzyme by heat-treatment at 90°C for 10 minutes, 100 g of a casein hydrolysate was obtained by lyophilizing the solution. The obtained casein hydrolysate had a degree of hydrolysis of 10.8% and a number-average molecular weight of 750 dalton.

Hereinafter, the present invention will be described in detail by showing Test Examples.

[Test Example 1]

The present test was carried out to detect cysteine protease inhibitory substance in milk.

(1) Detection method

The inventor used a technique "reverse zymography" as a method of detecting protease inhibitory substance and detected a protease inhibitory substance located on the gel of SDS-polyacrylamide gel electrophoresis. The reverse zymography is based on a technique opposite of the normal zymography, and the basic principle of the reverse zymography is as follows. That is, a sample containing protease inhibitory substance is applied onto SDS-polyacrylamide gel containing gelatin, and electrophoresis is performed, followed by soaking the gel in a protease solution to degrade protein in the gel. Protease activity is inhibited on a portion where an inhibitory substance is present and gelatin of the portion avoids being degraded by protease and is stained with staining solution, which enables detection of inhibitory substance.

(2) Test method

A method of the reverse zymography in the present invention is as follows.

Using total milk protein and natural bovine β-casein as samples, electrophoresis (hereinafter, SDS-polyacrylamide gel electrophoresis is sometimes abbreviated to SDS-PAGE) was conducted with 12.5% SDS-polyacrylamide gel containing 0.1% of gelatin. After electrophoresis, the gel was washed by immersing it in a 2. 5%Triton X-100 solution for 45 minutes, and further washed by repeating three times the operation of immersing the gel in distilled water for 45 minutes. Then, the gel was immersed in 100 ml of a 0. 025 M acetic acid buffer solution (pH 5. 5) containing 1 mg of papain (31units/ml), and gelatin was digested by keeping the solution at 37°C for 10 hours. The gel was washed with distilled water, stained with a staining solution (0.025% Coomassie brilliant blue (CBB) R-250, 40% methanol, 7% acetic acid aqueous solution) for one hour, and then destained with a destaining solution (40% methanol, 10% acetic acid aqueous solution).

Aside from this, as a control test, the reverse zymography was conducted with a 12.5% SDS-polyacrylamide gel not containing gelatin in the same way as above. Further, typical 12.5% SDS-PAGE (CBB staining) was also performed.

(3) Test results

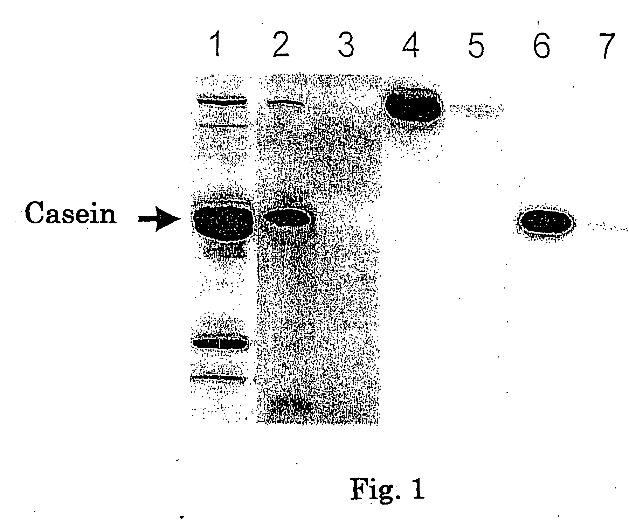

The results of the present test are as shown in Fig. 1. Fig. 1 shows the pattern of the reverse zymography. Lane 1 in Fig. 1 shows a typical SDS-PAGE pattern of the total protein in milk, lane 2 shows a pattern of the reverse zymography of the total protein in milk, lane 3 shows a pattern of the reverse zymography (control), in which the gel does not contain gelatin, of the total protein in milk, lane 6 shows a pattern of the reverse zymography of the natural bovine β-casein, and lane 7 shows a pattern of the reverse zymography (control), in which the gel does not contain gelatin, of the natural bovine β-casein, respectively. Arrows in the figures show a position of migration of the natural bovine β-casein (35 kDa in molecular weight) in SDS-PAGE. In addition, lanes 4 and 5 have no direct relations with the present test examples.

As is apparent from Fig. 1, a positive band of the reverse zymography was found in the position almost identical with the position of migration of bovine β-casein (35kDa) in lane 2. This ensured the presence of a substance having cysteine protease inhibitory activity in milk. In addition, a positive band was found in lane 6 which shows the reverse zymography using papain in which natural bovine β-casein was migrated.

The above-described results suggested that β-casein derived from bovine has cysteine protease inhibitory activity.

[Test Example 2]

The present test was carried out to determine the N-terminal amino acid sequence of a band of 35 kDa which was suggested to have cysteine protease inhibitory activity in Test Example 1.

(1) Test method

SDS-PAGE was carried out with total protein sample in milk used in Test Example 1, and proteins were transferred to polyvinylidene difluoride (PVDF) membrane. The PVDF membrane was stained with CBB, and then the stained band migrated in the vicinity of 35 kDa was cut out. The N-termini amino acid sequence of this band was determined with a G1005A Protein Sequencing System, manufactured by Hewlett-Packard Company.

(2)Test result

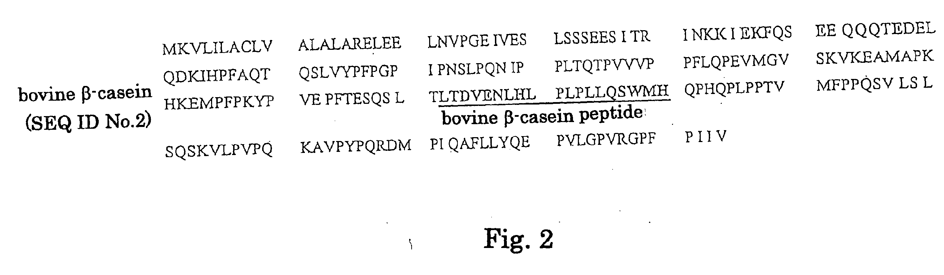

The result of the present test is as shown in Fig. 2. Fig. 2 shows the result of the determination of the amino acid sequence of 35 kDa stained band in milk. The result shows that the N-terminal amino acid sequence of a stained band of 35 kDa has completely matched with that of bovine β-casein. Accordingly, both results of the present test and Test Example 1 revealed that bovine β-casein has cysteine protease inhibitory activity.

[Test Example 3]

The present test was carried out to detect a region having cysteine protease inhibitory activity in a casein molecule.

(1) Test method

250 μg of bovine β-casein was dissolved in a 100 mM Tris-HCl buffer solution (pH 8.5), and digested with lysylendopeptidase at 35°C for 16 hours. Cysteine protease inhibitory activity was determined to confirm whether the digested bovine β-casein peptide mixture retains the cysteine protease inhibitory activity.

The inhibitory activity was measured with reference to the method by Barrett et al. ("Methods in Enzymology, " Vol. 80, p 535-561, 1981) as described below. Namely, Z-Phe-Arg-MCA (final concentration of 20 mM: manufactured by Peptide Institute, Inc.) was added as a substrate to a solution containing bovine β-casein peptide mixture dissolved in a 0.1 M acetic acid buffer solution pH 5.5. Then, a cysteine protease (papain in the present test: manufactured by Sigma Co., Ltd.) solution (final concentration: 15 units/ml) was added, the whole solution was mixed and the reaction was carried out at 37°C for 10 minutes. Subsequently, fluorescence intensity (excitation wavelength: 370 nm, emission wavelength: 460 nm) of AMC released from the digested substrate was measured with a fluorescence spectrometer (manufactured by Hitachi, Ltd.).

Since the measurement ensured that the bovine β-casein peptide mixture has the cysteine protease inhibitory activity, major peaks were separated from the peptide mixture with an acetonitrile linear concentration elution method based on reverse phase HPLC using TSK Gel DDS-80Ts column (manufactured b Tosoh Corporation). Then, cysteine protease inhibitory activity was measured for each sample of separated peaks (hereinafter, described as peptide samples) in the same way as above.

Amino acid sequences of peptide samples which had been shown to have an activity as a result of measurement of the inhibitory activity of the peptide samples were determined with a G1005A Protein Sequencing System, manufactured by Hewlett-Packard Company.

(2) Test result

The result of the present test is shown in Fig. 2. The result revealed that the major peptide sample having inhibitory activity has amino acid sequence (underlined portion: hereinafter, the peptide having the sequence is described as bovine β-casein peptide) ranging from Leu of 142th residue to His of 160th residue in the amino acid sequence of bovine β-casein in Fig. 2.

[Test Example 4]

The present test was carried out to measure an inhibitory activity of caseins on cysteine protease.

(1) Test method

Each of bovine β-casein, bovine α-casein and bovine κ-casein was used as a test sample. Cysteine protease-inhibitory activity of each test samples was measured in the same method as that of measuring the cysteine protease-inhibitory activity described in the Test Example 3.

(2) Test results

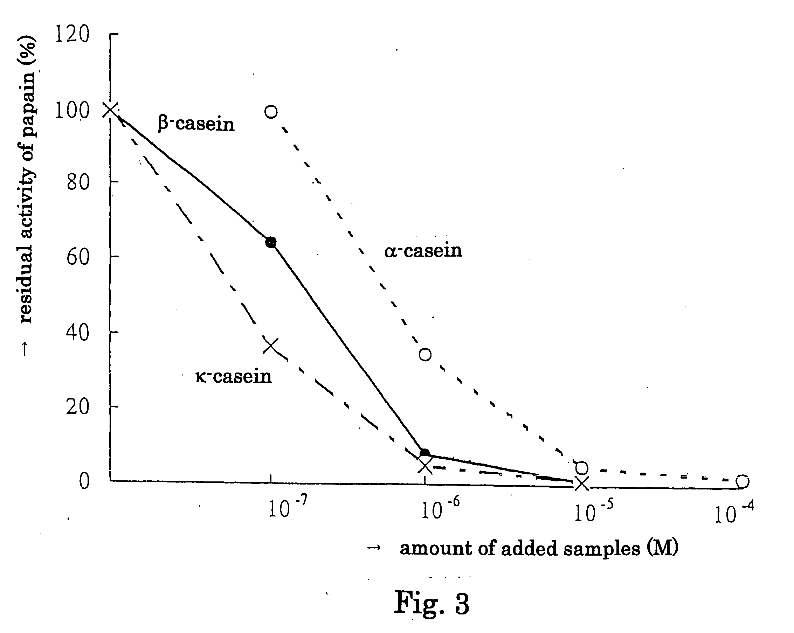

The results of the present test are as shown in Fig. 3. Fig. 3 shows cysteine protease-inhibitory activity of each of the bovine β-casein, bovine α-casein and bovine κ-casein to papain. The results revealed that β-casein and κ-casein completely inhibit papain at a concentration of $10^{-5}$ M, and that α-casein, though somewhat weak, almost inhibits papain activity at a concentration of $10^{-4}$ M. Accordingly, κ-casein and α-casein as well as β-casein were found to have the cysteine protease-inhibitory activity.

[Test Example 5]

The present test was carried out to measure a spectrum of cysteine protease-inhibitory activity of bovine β-casein.

(1) Test method

A spectrum of cysteine protease-inhibitory activity of a test sample was measured in the same way as that of measuring the cysteine protease-inhibitory activity described in the Test Example 3, using bovine β-casein as a test sample, and papain, cathepsin B and cathepsin L as cysteine proteases.

(2) Test results

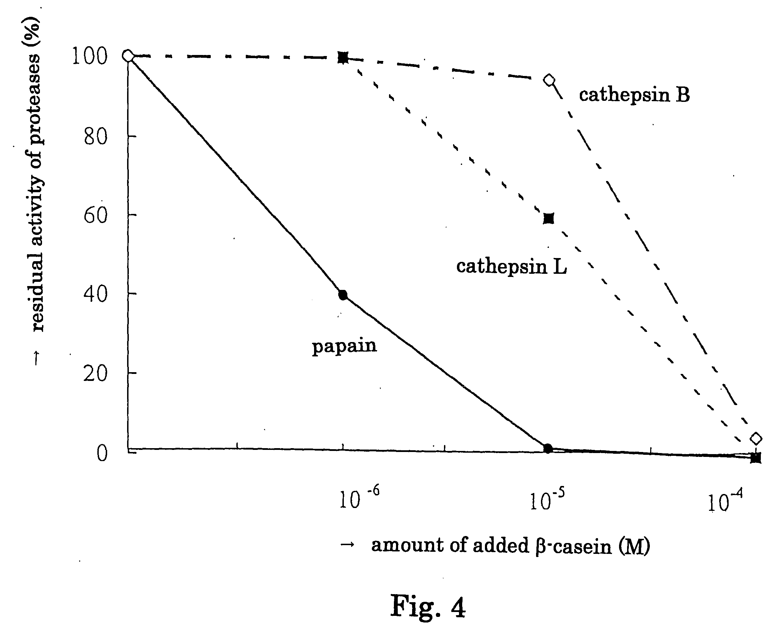

The results of the present test are as shown in Fig. 4. Fig. 4 shows the result of the measurement of an inhibitory activity of bovine β-casein to papain, cathepsin B and cathepsin L. The result revealed that bovine β-casein completely inhibits papain at a concentration of $10^{-5}$ M. It also revealed that bovine β-casein almost inhibits protease activities of cathepsin B and cathepsin L at a concentration of $10^{-4}$ M. Accordingly, it became apparent that bovine β-casein inhibits protease activities of papain, cathepsin B and cathepsin L, and that it has a wide spectrum of the cysteine protease-inhibitory activity.

[Test Example 6]

The present test was carried out to measure an inhibitory activity of human β-casein on cysteine protease.

(1) Test method

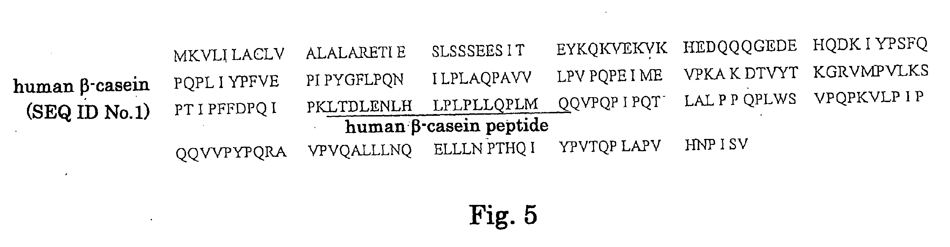

Cysteine protease-inhibitory activity of a test sample was measured in the same way as the method of measuring the cysteine protease-inhibitory activity described in Test Example 3, using a test sample of human β-casein purified according to a conventional procedure (for example, purified according to the method described in "J. Daily Sci." Vol. 53, No. 2, pp. 136 to 145, 1970), or a peptide having an amino acid sequence of amino acid numbers 133 to 151 of the amino acid sequence shown in SEQ ID No. 1 (amino acid sequence of human β-casein) of the Sequence Listing (underlined peptide of human β-casein in Fig. 5: hereinafter, the peptide having the sequence will be referred to as human β-casein peptide) which was synthesized in Example 1.

(2) Test results

The results of the present test revealed that human β-casein almost completely inhibits protease activity of papain at a concentration of $10^{-5}$ M, and that human β-casein peptide inhibits 65% of protease activity of papain at a concentration of $10^{-5}$ M and completely inhibits protease activity of papain at a concentration of $10^{-4}$ M.

[Test Example 7]

The present test was carried out to measure an inhibitory activity of a casein hydrolytsate to cysteine protease.

(1) Sample preparation

Casein hydrolysate was produced in the same way as in Production Example 2 except that 2,000, 8,000 or 9,000 units of pancreatin were added, and each hydrolysate produced thereby was named as a test sample 1, test sample 2 or test sample 3. The degree of hydrolysiss (%) of the test sample 1, test sample 2 and test sample 3 were 8.2, 33.5 and 38.0, respectively. In addition, the number-average molecular weights (dalton) of the test sample 1, test sample 2 and test sample 3 were 1,020, 250, and 210, respectively.

(2) Test method

Z-Phe-Arg-MCA (final concentration of 20 mM: manufactured by Peptide Institute, Inc.) was added as a substrate into a solution in which a test sample was dissolved in a 0.1 M acetic acid buffer (pH 5.5). Then, a papain solution (final concentration of 15 units/ml) as a cysteine protease solution was added and mixed, and the reaction was performed at 37°C for 10 minutes. Subsequently, fluorescence intensity (excitation wavelength: 370 nm, emission wavelength: 460 nm) of AMC released from the digested substrate was measured with a fluorescence spectrometer (manufactured by Hitachi, Ltd.).

(3) Test results

The results of the present test are as shown in Table. 1. Table 1 shows cysteine protease-inhibitory activity of each test sample. The results revealed that the test sample 1 inhibited 39% of cysteine protease activity of papain at a concentration of 0.1 mg/ml, and inhibited 61% at a concentration of 0.2 mg/ml. The results also revealed that test sample 2 inhibited 76% of cysteine protease activity of papain at a concentration of 0.2 mg/ml, and inhibited 50% at a concentration of 0.05 mg/ml, and that the test sample 3 inhibited 53% of cysteine protease activity of papain at a concentration of 0.2 mg/ml, 44% at a concentration of 0.1 mg/ml and 37% at a concentration of 0.05 mg/ml.

[Table 1]

| Sample | Degree of hydrolysis (%) | Number-average molecular weight (Dalton) | Inhibition rate at each concentration (%) | | |
|---|---|---|---|---|---|
| | | | 0.2 (mg/ml) | 0.1 (mg/ml) | 0.05 (mg/ml) |
| Test sample 1 | 8.2 | 1,020 | 61 | 39 | 8 |
| Test sample 2 | 33.5 | 250 | 76 | 64 | 50 |
| Test sample 3 | 38.0 | 210 | 53 | 44 | 37 |

Examples

Next, the present invention will be more specifically described by showing examples. However, the present invention is not limited to the following examples.

[Example 1]

(Preparation of a tablet in which bovine β-casein is formulated)

A tablet of a cysteine protease inhibitor having the following compositions was produced according to the following procedures.

Bovine β-casein (manufactured by Sigma Co., Ltd.) 40.0(%)
Lactose (manufactured by Morinaga Milk Industry Co., Ltd.) 18.5
Cornstarch (manufactured by Nisshin Flour Milling Co., Ltd.) 30.7
Magnesium stearate (manufactured by Taihei Chemical IndustrialCo., Ltd.) 1.4
Carboxymethyl cellulose calcium (manufactured by Gotoku Chemical Co., Ltd.) 9.4

A mixture of bovine β-casein, lactose, cornstarch and carboxymethyl cellulose calcium was kneaded uniformly while sterile purified water was appropriately added, and the kneaded product was dried at 50°C for three hours. Magnesium stearate was then added to the obtained dried product and mixed, followed by compression according to a conventional procedure, thereby a tablet was obtained.

[Example 2]

(Preparation of encapsulated bovine β-casein)

600 g of lactose (manufactured by Wako Pure Chemical Industries, Ltd.), 400 g of cornstarch (manufactured by Nisshin Flour Milling Co., Ltd.), 400 g of crystalline cellulose (manufactured by Wako Pure Chemical Industries, Ltd.) and 600 g of bovine β-casein (manufactured by Sigma Co., Ltd.) were sieved with 50 mesh sieve (manufactured by Yamato Scientific Co., Ltd.), and put into a polyethylene bag with a thickness of 0.5 mm to allow to mix upside down. The obtained powder was packed into a capsule (manufactured by Shionogi Qualicaps, Inc., gelatin capsule No. 1, Op Yellow No. 6 Body, 75 mg of empty weight) in a content of 275 mg using a fully automatic capsule filling machine (manufactured by Cesere Pedini, press type), thereby 7,000 capsules each containing 82 mg of bovine β-casein were obtained.

[Example 3]

(Preparation of drink in which bovine β-casein is added)

90 g of skim milk (manufactured by Morinaga Milk Industry Co., Ltd.) were dissolved in 800 ml of hot water at a temperature of 50°C, and 30 g of sugar (manufactured by Nissin Sugar Manufacturing Co., Ltd.), 14 g of instant coffee powder (manufactured by Nestle), 2 g of caramel (manufactured by Showa Kako Co., Ltd.) and 0.01 g of coffee flavor (manufactured by San-Ei Gen F.F.I. Inc.) were sequentially added and dissolved in the solution while stirring. Then, the mixture was cooled to 10°C, and 1 g of bovine β-casein (manufactured by Sigma Co., Ltd.) was added, thereby milk beverage containing about 0.1% of bovine β-casein and having cysteine protease-inhibitory activity was obtained.

[Example 4]

(Preparation of powder of enteral nutrition formula in which bovine β-casein is added)

10.8 kg of enzyme degradation product of whey protein (manufactured by Morinaga Milk Industry Co., Ltd.), 36 kg of dextrin (manufactured by Showa Sangyo Co., Ltd.), and a small amount of water-soluble vitamin and minerals were dissolved in 200 kg of water to prepare an aqueous phase in a tank. Aside from this, 3 kg of soybean salad oil (manufactured by Taiyo Yushi Co., Ltd.), 8.5 kg of palm oil (manufactured by Taiyo Yushi Co., Ltd.), 2.5 kg of safflower oil (manufactured by Taiyo Yushi Co., Ltd.), 0.2 kg of lecithin (manufactured by Ajinomoto Co., Inc.), 0.2 kg of fatty acid monoglyceride (manufactured by Kao Corporation) and a small amount of fat-soluble vitamin were mixed and dissolved to prepare an oil phase. The oil phase was added to the aqueous phase in the tank and mixed by stirring, followedbyheatingto70°Candhomogenized by a homogenizer under a pressure of 14.7 MPa. Next, the resultant was sterilized at 90°C for 10 minutes, concentrated and spray-dried, thereby approximately 59 kg of powder of intermediate product was obtained. Then, 6.8 kg of sucrose (manufactured by Hokuren-Federation of Agriculture Cooperatives), 167 g of amino acid mixture powder (Aj inomoto Co., Inc.) and 60 g of bovine β-casein (manufactured by Sigma Co. ,

Ltd.) were added to 50 kg of the powder of the intermediate product, and uniformly mixed, thereby approximately 57 kg of enteral nutrition formula powder containing bovine β-casein and having a cysteine protease-inhibitory activity was obtained.

[Example 5]

(Preparation of a tablet in which hydrolysate of bovine casein is formulated)

A tablet of cysteine protease inhibitor having the following composition was produced according to the following procedure.

    Casein hydrolysate produced in Production Example 2 40.0 (%)
    Lactose (manufactured by Morinaga Milk Industry Co., Ltd.) 18.5
    Cornstarch (manufactured by Nisshin Flour Milling Co., Ltd.) 30.7
    Magnesium stearate (manufactured by Taihei Chemical Industrial Co. , Ltd.) 1.4
    Carboxymethylcellulose calcium (manufactured by Gotoku Chemical Company Ltd.) 9.4

A mixture of bovine casein hydrolysate, lactose, cornstarch and carboxymethyl cellulose calcium was kneaded uniformly while sterile purified water was appropriately added, and the whole was dried at 50°C for three hours. Magnesium stearate was then added to the obtained dried product and mixed, followed by compression according to a conventional procedure, thereby a tablet was obtained.

[Example 6]

(Preparation of drink in which hydrolysate of bovine casein was added)

90 g of skim milk (manufactured by Morinaga Milk Industry Co., Ltd.) was dissolved in 800 ml of hot water at a temperature of 50°C, and 30 g of sugar (manufactured by Nissin Sugar Manufacturing Co. , Ltd.), 14 g of instant coffee powder, 2 g of caramel (manufactured by Showa Kako Co., Ltd.) and 0.01 g of coffee flavor (manufactured by San-Ei Gen F.F.I. Inc.) were sequentially added and dissolved while stirring. Then, the mixture was cooled to 10°C and 1 g of hydrolysate of bovine casein produced in Production Example 2 was added into the mixture, thereby milk beverage containing about 0.1% of hydrolysate of bovine β-casein and having a cysteine protease-inhibitory activity was obtained.

[Example 7]

(Preparation of enteral nutrition formula in which hydrolysate of bovine casein is added)

10.8 kg of hydrolyzed product of whey protein (manufactured by Morinaga Milk Industry Co., Ltd.), 36 kg of dextrin (manufactured by Showa Sangyo Co. , Ltd.), small amounts of water-soluble vitamin and mineral were dissolved in 200 kg of water to prepare an aqueous phase in a tank. Aside from this, 3 kg of soybean salad oil (manufactured by Taiyo Yushi Co., Ltd.), 8.5 kg of palm oil (available from Taiyo Yushi Co., Ltd.), 2.5 kg of safflower oil (manufactured by Taiyo Yushi Co., Ltd.), 0.2 kg of lecithin (manufactured by Ajinomoto Co., Inc.), 0.2 kg of fatty acid monoglyceride (manufactured by Kao Corporation) and a small amount of fat-soluble vitamin were mixed and dissolved to prepare an oil phase. The oil phase was added to the aqueous phase in the tank, and the whole was mixed by stirring, heated to 70°C and homogenized under a pressure of 14.7 MPa with a homogenizer. The resultant was sterilized at 90°C for 10 minutes, then concentrated and spray-dried, thereby approximately 59 kg of powder of intermediate product was prepared. 6.8 kg of sucrose (Hokuren-Federation of Agriculture Cooperatives), 167 g of powder of amino acid mixture (Ajinomoto Co., Inc.) and 60 g of hydrolysate of bovine casein produced in Production Example 2 were added to 50 kg of the powder of intermediate product, and the whole was mixed uniformly, thereby approximately 57 kg of enteral nutrition formula powder containing hydrolysate of bovine casein and having a cysteine protease-inhibitory activity was produced.

Industrial Applicability

As described in detail above, the present invention relates to a cysteine protease inhibitor comprising casein, a partial peptide of casein and/or hydrolysate of casein as an active ingredient. Effects exerted by the present invention are as follows:

(1) Excellent in safety and capable of being administrated or ingested daily in a long term because it is made of a protein available as food material, partial peptide thereof, and/or a hydrolysate thereof.
(2) Having a wide spectrum of inhibitory activity to various cysteine proteases.
(3) Usable as preventive or therapeutic agents for diseases associated with cysteine protease.
(4) Applicable to production of functional food including food for specified health uses and nutritional supplementary food.
(5) Usable as an agent for adjusting physical properties of food in the field of food processing.

**Claims**

1. A cysteine protease inhibitor comprising casein or a partial peptide thereof as an active ingredient.

2. The cysteine protease inhibitor according to claim 1, wherein the casein or the partial peptide thereof is derived from human or bovine.

3. A cysteine protease inhibitor comprising casein as shown in the following (A) or (B) or a partial peptide thereof as an active ingredient;

   (A) a peptide having amino acid sequence of at least amino acid numbers 133 to 151 of the amino acid sequence shown in SEQ ID No. 1 of the Sequence Listing, or
   (B) a peptide having amino acid sequence of at least amino acid numbers 133 to 151 of the amino acid sequence shown in the SEQ ID No. 1 of the Sequence Listing, including substitution, deletion, insertion, addition or inversion of one or plural amino acids, and having cysteine protease inhibitory activity.

4. A cysteine protease inhibitor containing casein as shown in the following (C) or (D) or a partial peptide thereof as an active ingredient;

   (C) a peptide having amino acid sequence of at least amino acid numbers 142 to 160 of the amino acid sequence shown in SEQ ID No. 2 of the Sequence Listing, or
   (D) a peptide having amino acid sequence of at least amino acid numbers 142 to 160 of the amino acid sequence shown in the SEQ ID No. 2 of the Sequence Listing, including substitution, deletion, insertion, addition or inversion of one or plural amino acids, and having cysteine protease inhibitory activity.

5. A cysteine protease inhibitor, comprising casein hydrolysate which is obtainable by hydrolyzing casein with protease and has cysteine protease inhibitory action, as an active ingredient.

6. The cysteine protease inhibitor according to claim 5, wherein the protease is one or a plurality of proteases selected from the group consisting of proteases derived from animals and proteases derived from microorganisms.

7. The cysteine protease inhibitor according to claim 5 or 6, wherein degree of hydrolysis of the casein hydrolysate is 6 to 45%.

8. The cysteine protease inhibitor according to any one of claims 5 to 7, wherein number-average molecular weight of the casein hydrolysate is 200 to 5,000 dalton.

9. The cysteine protease inhibitor according to any one of claims 5 to 8, which comprises casein hydrolysate not less than 0.005% by mass with respect to the total amount.

10. The cysteine protease inhibitor according to any one of claims 1 to 9, wherein the cysteine protease inhibitor is a preventive or therapeutic agent for a disease associated with cysteine protease.

11. The cysteine protease inhibitor according to claim 10, wherein the disease associated with the cysteine protease is osteoporosis, malignant hypercalcemia, breast cancer, prostate cancer, periodontitis or bacterial and viral infectious diseases.

12. A food and drink composition or feed composition, which is produced by adding the cysteine protease inhibitor according to any one of claims 1 to 11.

13. A method for treating a disease associated with cysteine protease, wherein the cysteine protease inhibitor according to any one of claims 1 to 11 is administered to a subject.

14. A use of casein or a partial peptide thereof in manufacture of a cysteine protease inhibitor.

15. The use according to claim 14, wherein the casein or the partial peptide thereof is derived from human or bovine.

16. A use of casein as shown in the following (A) or (B) or a partial peptide thereof in manufacture of a cysteine protease inhibitor;

    (A) a peptide having amino acid sequence of at least amino acid numbers 133 to 151 of the amino acid sequence shown in SEQ ID No. 1 of the Sequence Listing, or
    (B) a peptide having amino acid sequence of at least amino acid numbers 133 to 151 of the amino acid sequence shown in SEQ ID No. 1 of the Sequence Listing, including substitution, deletion, insertion, addition or inversion of one or plural amino acids, and having cysteine protease inhibitory activity.

17. A use of casein as shown in the following (C) or (D) or a partial peptide thereof in manufacture of a cysteine protease inhibitor;

    (C) a peptide having amino acid sequence of at least amino acid numbers 142 to 160 of the amino acid sequence shown in SEQ ID No. 2 of the Sequence Listing, or
    (D) a peptide having amino acid sequence of at least amino acid numbers 142 to 160 of the amino acid sequence shown in SEQ ID No. 2 of the Sequence Listing, including substitution, deletion, insertion, addition or inversion of one or plural amino acids, and having cysteine protease inhibitory activity.

18. A use of a casein hydrolysate which is obtainable by hydrolyzing casein with a protease and has cysteine protease inhibitory action, in manufacture of a cysteine protease inhibitor.

19. The use according to claim 18, wherein the protease is one or a plurality of proteases selected from the group consisting of proteases derived from animals and proteases derived from microorganisms.

20. The use according to claim 18 or 19, wherein degree of hydrolysis of the casein hydrolysate is 6 to 45%.

21. The use according to any one of claims 18 to 20, wherein number-average molecular weight of the casein hydrolysate is 200 to 5,000 dalton.

22. The use according to any one of claims 18 to 21, which comprises casein hydrolysate not less than 0. 005% by mass with respect to the total amount.

23. The use according to any one of claims 14 to 22, wherein the cysteine protease inhibitor is a preventive or therapeutic agent for a disease associated with cysteine protease.

24. The use according to claim 23, wherein the disease associated with cysteine protease is osteoporosis, malignant hypercalcemia, breast cancer, prostate cancer, periodontitis or bacterial and viral infectious diseases.

Fig. 1

bovine β-casein
(SEQ ID No.2)

MKVLILACLV ALALARELEE LNVPGE IVES LSSSEES I TR I NKK I EKFQS EE QQQTEDEL

QDKIHPFAQT QSLVYPFPGP I PNSLPQN IP PLTQTPVVVP PFLQPEVMGV SKVKEAMAPK

HKEMPFPKYP VE PFTESQS L TLTDVENLHL PLPLLQSWMH QPHQPLPPTV MFPPQSV LS L

bovine β-casein peptide

SQSKVLPVPQ KAVPYPQRDM PI QAFLLYQE PVLGPVRGPF P I I V

Fig. 2

Fig. 3

Fig. 4

human β-casein
(SEQ ID No.1)

MKVLI LACLV    ALALARETI E    SLSSSEES I T    EYKQKVEKVK    HEDQQQGEDE    HQDK I YPSFQ

PQPL I YPFVE    PI PYGFLPQN    I LPLAQPAVV    LPV PQPE I ME    VPKA K DTVYT    KGRVMPVLKS

PT I PFFDPQ I    PKLTDLENLH    LPLPLLQPLM    QQVPQP I PQT    LAL P P QPLWS    VPQPKVLP I P

human β-casein peptide

QQVVPYPQRA    VPVQALLLNQ    ELLLN PTHQ I    YPVTQP LAPV    HNP I SV

**Fig. 5**

EP 1 568 377 A1

# INTERNATIONAL SEARCH REPORT

| International application No. |
|---|
| PCT/JP03/14263 |

## A. CLASSIFICATION OF SUBJECT MATTER
Int.Cl$^7$  A61K38/57, A61P1/02, 3/14, 19/10, 31/04, 31/12, 35/00, 43/00

According to International Patent Classification (IPC) or to both national classification and IPC

## B. FIELDS SEARCHED

Minimum documentation searched (classification system followed by classification symbols)
Int.Cl$^7$  A61K38/00-38/58, 45/00-45/08, A61P1/00-43/00

Documentation searched other than minimum documentation to the extent that such documents are included in the fields searched

Electronic data base consulted during the international search (name of data base and, where practicable, search terms used)
DDBJ/GanBank/EMBL, SwissProt/PIR/PDB, GeneSeq, MEDLINE(STN), EMBASE(STN), BIOSIS(STN), BIOTECHABS(STN), CAPLUS(STN), WPI(DIALOG), JSTPLUS(JOIS), JMEDPLUS(JOIS)

## C. DOCUMENTS CONSIDERED TO BE RELEVANT

| Category* | Citation of document, with indication, where appropriate, of the relevant passages | Relevant to claim No. |
|---|---|---|
| X<br><br>Y<br>A | LEE, H.S., LEE, K.J. Cathepsin B inhibitory peptides derived from β-casein. Peptides, 2000, 21, pages 807 to 809; full text | 1,2,5,6,7-9,<br>14,15,18-20,<br>21,22<br>10-12,23,24<br>3,4,16,17 |
| Y | EP 679659 A1  (TAIHO PHARMACEUTICAL CO., LTD.), 02 November, 1995 (02.11.95), Full text<br>& JP 7-179496 A        & WO 95/13302 A1<br>& AU 9480674 A         & NO 9502748 A<br>& US 5698519 A | 10-12,23,24 |
| Y | JP 7-242600 A  (Yoshimitsu NAGAO), 19 September, 1995 (19.09.95), Full text (Family: none) | 10-12,23,24 |

☒ Further documents are listed in the continuation of Box C. ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 15 December, 2003 (15.12.03) | 13 January, 2004 (13.01.04) |

| Name and mailing address of the ISA/<br>Japanese Patent Office | Authorized officer |
|---|---|
| Facsimile No. | Telephone No. |

Form PCT/ISA/210 (second sheet) (July 1998)

**INTERNATIONAL SEARCH REPORT**

| International application No. |
| --- |
| PCT/JP03/14263 |

C (Continuation).  DOCUMENTS CONSIDERED TO BE RELEVANT

| Category* | Citation of document, with indication, where appropriate, of the relevant passages | Relevant to claim No. |
| --- | --- | --- |
| Y | JP 9-221425 A  (Taiho Pharmaceutical Co., Ltd.), 26 August, 1997 (26.08.97), Full text (Family: none) | 10-12,23,24 |
| Y | WO 98/49152 A1  (SMITHKLINE BEECHAM CORP.), 05 November, 1998 (05.11.98), Claims & JP 2002-501502 A      & ZA 9803479 A & AU 98713995 A      & EP 977743 A1 | 10-12,23,24 |
| Y | JP 2001-139534 A  (Yoshimitsu NAGAO), 22 May, 2001 (22.05.01), Full text (Family: none) | 10-12,23,24 |
| Y | JP 2000-72797 A  (Taiho Pharmaceutical Co., Ltd.), 07 March, 2000 (07.03.00), Claims (Family: none) | 10-12,23,24 |
| Y | EP 822260 A1  (Taiho Pharmaceutical Co., Ltd.), 04 February, 1998 (04.02.98), Claims & WO 97/31122 A1          & AU 9717340 A & US 5914348 A          & KR 99007955 A | 10-12,23,24 |
| Y | JP 7-2896 A  (Snow Brand Milk Products Co., Ltd.), 06 January, 1995 (06.01.95), Full text (Family: none) | 10-12,23,24 |
| Y | JP 7-126294 A  (Snow Brand Milk Products Co., Ltd.), 16 May, 1995 (16.05.95), Full text (Family: none) | 10-12,23,24 |
| Y | MATSUOKA, Y. et al., Cystatin C in Milk Basic Protein (MBP) and Its Inhibitory Effect on Bone Resorption in Vitro., Biosci.Biotechnol.Biochem., 2002, 66(12), pages 2531 to 2536; particularly, summary | 10-12,23,24 |
| Y A | Beta casein precursor.[online].SWISS-PROT, 1988. [retrieved on 2003-12-12]. Retrieved from JPO DNA Database. Accession No. PO5814 | 2,15 1,3-12,14, 16-24 |
| A | Beta casein precursor.[online].SWISS-PROT, 1986. [retrieved on 2003-12-12]. Retrieved from JPO DNA Database. Accession No. PO2666 | 1-12,14-24 |

Form PCT/ISA/210 (continuation of second sheet) (July 1998)

## INTERNATIONAL SEARCH REPORT

International application No.

PCT/JP03/14263

| C (Continuation). DOCUMENTS CONSIDERED TO BE RELEVANT | | |
|---|---|---|
| Category* | Citation of document, with indication, where appropriate, of the relevant passages | Relevant to claim No. |
| L | JP 5-184382 A (Kyodo Nyugyo Kabushiki Kaisha), 27 July, 1993 (27.07.93), (Family: none) (It is disclosed in this document that activity of αs-, β- and κ-casein as cysteine protease inhibitor is 0%.) | 1-12,14-24 |
| L | SUZUKI, J., KATOH, N., Cysteins Protease in Bovine Milk Capable of Hydrolyzing Casein as the Substrate and Elevation of the Activity during the Course of Mastitis., Jpn.J.Vet.Sci., 1990, 52(5), pages 947 to 954 (It is disclosed in this document that casein is hydrolyzed by cysteine protease.) | 1-12,14-24 |
| A | JP 8-81388 A (Morinaga Milk Industry Co., Ltd.), 26 March, 1996 (26.03.96), (Family: none) | 1-12,14-24 |
| A | EP 1040833 A1 (SNOW BRAND PRODUCTS, CO., LTD.), 04 October, 2000 (04.10.00), & JP 2000-281587 A & NZ 503608 A & AU 200022633 A | 1-12,14-24 |
| A | WO 95/32728 A1 (ABBOTT LABORATORIES), 07 December, 1995 (07.12.95), & JP 10-500101 A & EP 760673 A & AU 9521293 A & US 5643880 A & US 5707968 A & MX 9605829 A1 | 1-12,14-24 |
| A | WO 95/32727 A1 (ABBOTT LABORATORIES), 07 December, 1995 (07.12.95), & JP 10-500100 A & EP 760674 A1 & AU 9521920 A & US 5506209 A & US 5538952 A & BR 1101102 A3 & MX 9605830 A1 & NZ 329311 A | 1-12,14-24 |
| A | JP 6-128287 A (Nisshin Flour Milling Co., Ltd.), 10 May, 1994 (10.05.94), (Family: none) | 1-12,14-24 |
| P,X | OHASHI, A. et al., New functions of lactoferrin and β-casein in mammalian milk as cysteine protease inhibitors., Biochem.Biophys.Res.Commun., 2003, 306, pages 98 to 103; full text | 1-12,14-24 |

Form PCT/ISA/210 (continuation of second sheet) (July 1998)

**EP 1 568 377 A1**

**INTERNATIONAL SEARCH REPORT**

| International application No. |
| --- |
| PCT/JP03/14263 |

**Box I    Observations where certain claims were found unsearchable (Continuation of item 2 of first sheet)**

This international search report has not been established in respect of certain claims under Article 17(2)(a) for the following reasons:

1. ☒ Claims Nos.: 13

   because they relate to subject matter not required to be searched by this Authority, namely:
   The inventions as set forth in claim 13 pertains to methods for treatment of the human body by therapy.   (Article 17(2)(a)(i) of the PCT and Rule 39.1(iv) of the Regulations under the PCT)

2. ☐ Claims Nos.:

   because they relate to parts of the international application that do not comply with the prescribed requirements to such an extent that no meaningful international search can be carried out, specifically:

3. ☐ Claims Nos.:

   because they are dependent claims and are not drafted in accordance with the second and third sentences of Rule 6.4(a).

**Box II    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**      ☐      The additional search fees were accompanied by the applicant's protest.

☐      No protest accompanied the payment of additional search fees.

Form PCT/ISA/210 (continuation of first sheet (1)) (July 1998)

26